Europäisches Patentamt

(19) **European Patent Office**

**Office européen des brevets**

(11) Publication number: **0 040 590**
**A2**

(12) # EUROPEAN PATENT APPLICATION

(21) Application number: **81850087.8**

(22) Date of filing: **19.05.81**

(51) Int. Cl.³: **A 61 K 9/22**
**A 61 K 9/32**

(30) Priority: **21.05.80 SE 8003805**

(43) Date of publication of application:
**25.11.81 Bulletin 81/47**

(84) Designated Contracting States:
**AT BE CH DE FR GB IT LI LU NL SE**

(71) Applicant: **Aktiebolaget Hässle**

**S-431 83 Mölndal(SE)**

(72) Inventor: **Bogentoft, Conny Börje**
**Bäckvägen 12**
**S-430 50 Källered(SE)**

(74) Representative: **Wurm, Bengt Runio et al,**
**Patent and Trade Mark Department Ab Astra**
**S-151 85 Södertälje(SE)**

(54) **A pharmaceutical preparation having improved release properties.**

(57) An oral pharmaceutical preparation comprises a core containing a therapeutically active substance and a coating and is characterized in that the coating comprises an anionic carboxylic acrylic polymer soluble only above pH 5.5 in an amount of 10 to 85 per cent by weight of the coating and a waterinsoluble polymer selected from quaternary ammonium substituted acrylic polymers. The preparation enables release of a major part of the drug contents thereof in the lower part of the intestinal system.

EP 0 040 590 A2

Croydon Printing Company Ltd.

A pharmaceutical preparation having improved release
properties

## Description

### Technical field

The present invention is related to a pharmaceutical preparation for oral administration, having improved release
properties, to a method for preparing such preparation and
to a method of obtaining an improved profile of release of
a drug in the body of an animal or man.

The object of the invention is to obtain a preparation which
releases a major part of its drug contents in the lower part
of the intestinal system preferentially in the large intestine i.e. colon.

### Background art

The present knowledge of control of the release properties
in an oral pharmaceutical preparation is great and rapidly
growing. Especially the use of coatings in order to obtain
a desired release rate or release at a desired location in
the gastro-intestinal system has been described in a number
of publications among which US Patents 2 858 252, 2 853 420
and 3 835 221 could be mentioned as illustrative examples.
A number of preparations including an enteric coating on
tablets or small granular bodies are thus known.

In certain disease conditions it is strongly desirable to
obtain drug release mainly in the large intestine or in the
lower fourth of the small intestine. This is the case e.g.
with drugs used in the treatment of inflammatory conditions in the colon or lower ileum e.g. Colitis ulcerosa or Morbus Crohn. Another therapeutic area where such release properties are desired is the removal of excessive amounts of bile acid in the colon using e.g. an anion exchanger such as cholestyramine. However, no preparation providing the desirable release properties is available.

In U. S. Patent No. 3,957,523 a preparation with a coating composition is described, which has the object of providing a controlled intestinal release of a drug. Said coating composition comprises a mixture of an enterosoluble cellulose derivative having monoester linkages with a polybasic acid and a digestive fluid-insoluble cellulose derivative. In the case that one or both the two components are selected from vinyl polymers and acrylic polymers the intended object of the invention thus known is stated not to be obtainable.

U. S. Patent No. 3,431,338 describes a pharmaceutical composition comprising (1) a medicament-containing nucleus, coated sequentially with (2) a layer of an acid-soluble coating material which is resistant both to alkalis and intestinal juices, (3) a water-soluble intermediate layer, and (4) a layer of an alkali soluble coating material which is resistant to acid and gastric juices. Such composition is said to enable release of a drug such as emetine or dehydroemetine in the lower sections of the small intestine and the colon, as concluded from release tests on coated tablets. However, such sequential build-up of a coating is technically highly inconvenient and apt to give a product with unreliable properties.

## Disclosure_of_Invention

According to the present invention it has been found that by providing a core containing a therapeutically active substance with a coating of a specified composition the release of the active substance can be directed to the colon or the lower part of the ileum or both as desired, while disadvantages of previously known preparations are avoided.

Thus, the present invention provides an oral pharmaceutical preparation comprising a core containing a therapeutically active substance and a coating, characterized in that the coating comprises an anionic carboxylic acrylic polymer soluble only above pH 5.5 in an amount of 10 to 85 per cent by weight of the coating and a waterinsoluble polymer selected from quarternary ammonium substituted acrylic polymers in an amount of 15 to 90 per cent by weight of the coating. The coating normally has a thickness of 3-60 μm, preferably 10-30 μm.

The core may contain the active substance in admixture with carrier material or on the surface of a carrier particle, or the core may be made up of active substance only.

By an anionic polymer is meant a polymer containing anionic groups. For the purpose of this invention such polymer should be soluble above pH 5.5. Preferably the anionic carboxylic acrylic polymer is selected from partly methyl esterified methacrylic acid polymers.

Suitable waterinsoluble quarternary ammonium substituted acrylic polymers are polymers sold under the names Eudragit RS and Eudragit RL, having an ammonium substitution of about 5 and about 10 per cent by weight, respectively.

Suitable partly methyl esterified methacrylic acid
polymers are sold under the names Eudragit L and Eudragit S.

According to a preferred embodiment of the invention the
coating composition additionally comprises a plasticizer
selected from fatty alcohols or fatty acids. Especially
suitable plasticizers are cetanol (cetyl alcohol) and
stearic acid. The amount of plasticizer is suitably up to
25 per cent by weight of the coating.

Preferred therapeutically active substances to be included
in a pharmaceutical preparation of the invention are
salicylazo-sulfapyridine, 5-aminosalicylic acid and
cholestyramine, as well as salts and related compounds
having similar therapeutic properties.

The coated cores of the invention are to be included in
dosage units. Normally each dosage unit contains at least
about 10 bodies. Preferably the minimum number of bodies is
about 200. Among suitable dosage units tablets and capsules
are specifically mentioned. Pharmaceutically acceptable
additives may be included in the dosage units together with
the preparation of the invention.

The cores of the bodies of the preparation may include
pharmaceutically indifferent materials of the type normally
used in pharmaceutical preparations, such as polysaccharides,
microcrystalline cellulose, starch and waxes. The size of
the cores may be sieve fractions between 0.1 and 3.0 mm,
preferably between 0.5 and 1.5 mm.

Another aspect of the present invention is a process for preparing a pharmaceutical preparation as defined above, which process is characterized in that a large number of cores as defined above are provided with a coating as defined above by applying a solution of the coating composition to the cores. The coating is preferably applied by spraying the coating solution.

The solvents employed according to the process of the invention are solvents having a sufficient volatility to evaporate under the conditions of application, leaving a layer of the solute on the surface of the core or body prepared. Preferably organic solvents such as alcohols, hydrocarbons and esters are used as well as derivatives thereof, such as chlorinated hydrocarbons. The process of applying the coating may be carried out in an apparatus normally used in the pharmaceutical industry for coating of solid pharmaceutical preparations, preferably in a fluid bed apparatus. The process is normally carried out at ambient conditions, however, temperature and pressure conditions may be varied within broad limits. In a fluid bed spraying process the temperature of the inlet air is suitably 15 to $60^{\circ}$C.

Furhter aspects of the invention are a coating composition as defined above and a method of obtaining release of a therapeutically active substance in the lower part of the intestinal system and a method of treatment, both employing the preparation defined above.

The invention takes advantage of the fact that the time of passage through the small intestine is rather constant even in disease conditions connected with diarrhoea or obstipation. Thus, in man said time is 2 to 5 hours. According to the invention the change of pH from acid to about neutral at the pylorus is employed as a trigger mechanism changing the physical condition of the coating and finally causing release of the active substance after a predetermined lag time.

## Experimental

### Tests on release properties

As a model substance for study of the release properties salicylazo-sulfapyridine (SASP) was used. SASP is absorbed to a small extent in the stomach and the small intestine. In the colon it is completely broken down by bacteria to sulfapyridine (SP) and 5-amino-salicylic acid (5-ASA) which are then absorbed.

With a conventional preparation of SASP about 5 % of the dosage is excreted as SASP in the urine due to absorbtion in the small intestine. The total excreted amounts of SP and 5-ASA are about 75 % and 25 % respectively. With a colon release preparation the excreted amount of SASP would be strongly reduced while the excreted amounts of SP and 5-ASA would be substantially unchanged or increased.

### Preparation

The test preparation were prepared as follows:

SASP (2000 g), lactose (1960 g), cetanol (120 g), were mixed for 10 minutes in a planet mixer. Water (1000 g) containing 25 g carboxymethyl cellulose was then added slowly for 2 minutes whereupon the mixing continued for 10 minutes. The wet mixture was then extruded and spheronized. The granules were then dried at 50$^{\circ}$C for 1 hour in a fluid bed. The cores had the following sieve analysis:

| Sieve size mm | Per cent w/w |
|---|---|
| > 1.2 | 23 |
| 1.2 - 1.0 | 67 |
| 1.0 - 0.75 | 10 |
| < 0.75 | - |

## Tests in vitro

The rate of release of SASP was determined by the beaker method (Levy et al., New England Journal of Medicine, vol. 262, p. 1053-1058 (1960)) at 37°C and 100 rpm in phosphate buffers having pH 6.7 and 7.0. Samples were taken at different times and the amount of SASP released was determined spectrophotometrically. The cores were coated by airborne spraying. 500 g portions of granules were coated with each of the following coating solutions according to the present invention:

|  | Example 1 | Example 2 |
|---|---|---|
| Eudragit RS 100 | 30 | 22 |
| Eudragit S 100 | - | 13 |
| Eudragit L 100 | 30 | 7 |
| Cetanol | 22.5 | 15 |
| Methylene chloride | 750 | 500 |
| Isopropanol | 750 | 500 |

Release tests were made by the same method as with the core material. A conventional tablet preparation (Salazopyrin®) was also studied. The results of the release tests are given below.

Per cent SASP released at pH 6.7

| Time (h) | 1 | 2 | 4 | 6 | 8 | 12 | 16 |
|---|---|---|---|---|---|---|---|
| Preparation | | | | | | | |
| Core | 47 | 65 | 85 | 97 | | | |
| Tablet | 68 | 80 | 92 | | | | |
| Example 1 | 0 | 0 | 3 | 28 | 50 | 76 | 88 |
| Example 2 | 0 | 0 | 0 | 0 | 0 | 1 | 4 |

Per cent released at pH 7.0

| Time (h) | 1 | 2 | 4 | 6 | 8 |
|---|---|---|---|---|---|
| Preparation | | | | | |
| Core | 53 | 77 | 94 | | |
| Tablet | 86 | 91 | 95 | | |
| Example 1 | 0 | 17 | 63 | 83 | 93 |
| Example 2 | 0 | 0 | 30 | 69 | 89 |

Tests in vivo

Seven healthy voluntary test persons with an average age of 34 years (26-54) participated. The tablet and the two preparations of the invention described above were tested. The granular preparations of the invention were administered in a hard gelatine capsule. All preparations were given in one dose, randomized with a cross-over technique. Totally a dosage of 2 g SASP of each preparation was given, i.e. 4 tablets or 7 capsules of each granular preparation.

After at least 10 hours fasting the test preparation was ingested with 200 ml water. Three hours later restrictions on feeding were lifted. All urine was collected for 4 days. At least 7 days passed between each test period. The urine samples were stored deep-frozen before analysis. All urine samples were analyzed for SASP and acetyl-5-ASA by liquid chromatography methods. The latter substance, which is a metabolite of 5-ASA, was chosen for analysis as largely representing the total excretion of 5-ASA.

Statistic significance ($p < 0.05$) was computed by paired t-test and Wilcoxon test.

Amounts excreted in urine of SASP and acetyl-5-ASA is shown in the table below with mean values and SEM (standard error of the means).

Amounts of SASP and acetyl-5-ASA excreted in urine during 4 days after administration of 2 g of SASP:

| Preparation | SASP | | Acetyl-5-ASA | |
|---|---|---|---|---|
| | $\bar{M} \pm$ SEM ($\mu$mole) | % of dose | $\bar{M} \pm$ SEM ($\mu$mole) | % of dose |
| Tablet | 99 ± 15 | 2.0 | 1225 ± 106 | 24.5 |
| Example 1 | 37 ± 25 | 0.7 | 922 ± 120 | 18.4 |
| Example 2 | 24 ± 18 | 0.5 | 1187 ± 146 | 23.7 |

## Comments

The preparations of the invention according to Example 1 and Example 2 gave a significantly lowered amount of excreted SASP as compared to the tablet. Between said two preparations of the invention no significant difference was found. The excreted amounts of SASP in urine decrease with decreasing release rate in vitro. The amounts excreted of acetyl-5-ASA was not significantly different between the three preparations tested.

The results confirm that a preparation giving the desired
release properties is obtainable by the present invention.

The invention is further illustrated by examples and
release tests according to the beaker method given in the
table below.

| Example No | Active substance in core | Coating — Components (g) to 500 g of granules | | | | | Release test | | | | | | | | |
| | | Waterinsoluble polymer | | Anionic carboxylic acrylic polymer | | Plasticizer | | Amount (%) of active substance released after: | | | | | | | |
| | | Eudragit RS 100 | Eudragit RL 100 | Eudragit L 100 | Eudragit S 100 | Cetanol | pH | 1 | 2 | 4 | 6 | 8 | 12 | 16 h | |
|---|---|---|---|---|---|---|---|---|---|---|---|---|---|---|---|
| 3 | Salicylazosulfapyridine 1) | | 25 | 25 | | 5 | 6.5 | <1 | 6 | 37 | 61 | 76 | 92 | | |
| 4 | " | 25 | | 25 | | 5 | 6.5 | 0 | 6 | 38 | 69 | 84 | 98 | | |
| 5 | " | 20 | | 5 | | 2.5 | 6.7 | <1 | <1 | 3 | 13 | 35 | 71 | 90 | |
| 6 | Acetylsalicylic acid 2) | 20 | | 20 | | 15 | 6.5 | 2 | 6 | 23 | 46 | | | | |
| 7 | 5-Aminosalicylic acid 3) | 17.5 | | 7.5 | | 2.5 | 6.7 | <1 | 2 | 10 | 28 | 51 | 93 | | |

0040590

## Footnotes

1) Cores had a composition and were prepared as described under "Tests on release properties" above.

2) Compressed 100 % acetylsalicylic acid granules (ASA-Gran, Monsanto).

3) Cores in Example 7 consisted of 75 % active substance, 25 % cellulose avicel and 5 % poloxalene (Pluronic©). The cores were prepared by the process referred to under 1).

## Best mode of carrying out the invention

The best mode of carrying out the invention is the embodiment according to Example 7 above.

## Claims

1. An oral pharmaceutical preparation comprising a core containing a therapeutically active substance and a coating, characterized in that the coating comprises an anionic carboxylic acrylic polymer soluble only above pH 5.5 in an amount of 10 to 85 per cent by weight of the coating and a waterinsoluble polymer selected from quarternary ammonium substituted acrylic polymers in an amount of 15 to 90 per cent by weight of the coating.

2. A preparation according to claim 1, characterized in that the anionic carboxylic acrylic polymer is selected from partly methyl esterified methacrylic acid polymers.

3. A preparation according to one or more of the preceding claims, characterized in that the coating composition additionally comprises a plasticizer selected from fatty alcohols or fatty acids.

4. A process for preparing an oral pharmaceutical preparation comprising a core containing a therapeutically active substance and a coating, characterized in that a large number of cores are coated with a composition comprising an anionic carboxylic acrylic polymer soluble only above pH 5.5 in an amount of 10 to 85 per cent by weight and a waterinsoluble polymer selected from quarternary ammonium substituted acrylic polymers in an amount of 15 to 90 per cent of the coating, by applying a solution of the composition to the cores.

5. A coating composition characterized in that the dry substance thereof comprises an anionic carboxylic acrylic polymer soluble only above pH 5.5 in an amount of 10 to 85 per cent by weight and a waterinsoluble polymer selected from quarternary ammonium substituted acrylic polymers in an amount of 15 to 90 per cent by weight of the coating.

6. A method of obtaining release of a therapeutically active substance in the lower part of the intestinal system of an animal or man, characterized in administering orally to such host a preparation according to claim 1.

7. A method of treatment of inflammatory conditions in. the colon or lower ileum in an animal or man, characterized in administering orally to a host suffering from such conditions an effective amount of a preparation according to claim 1.

8. An oral pharmaceutical preparation, a process, a coating composition, a method of obtaining release or a method of treatment as claimed in one or more of claims 1-7 and substantially as described.